# EUROPÄISCHE PATENTANMELDUNG

(11) **EP 1 083 225 A1**
(43) Veröffentlichungstag der Anmeldung: **14.03.2001**
(21) Anmeldenummer: 00118935.6
(22) Anmeldetag: 01.09.2000
(51) Int. Cl.: C12N 15/52, C12P 13/02, C12N 9/00, C12N 1/21

(54) **Verfahren zur fermentativen Herstellung von D-Pantothensäure unter Verwendung coryneformer Bakterien**

(30) Priorität: 09.09.1999 DE 19943055; 30.06.2000 DE 10031999
(71) Anmelder: Degussa-Hüls Aktiengesellschaft, 60287 Frankfurt am Main (DE); Institut für Innovation an der Universität Bielefeld GmbH, 33615 Bielefeld (DE)
(72) Erfinder: Dusch, Nicole, Dr., 33619 Bielefeld (DE); Thierbach, Georg, Dr., 33613 Bielefeld (DE)

(57) **Zusammenfassung**

Die Erfindung betrifft ein Verfahren zur Herstellung von D-Pantothensäure durch Fermentation coryneformer Bakterien, bei dem man Bakterien einsetzt, in denen man die für die Pyruvat-Carboxylase (EC-Nummer 6.4.1.1.) codierende Nucleotidsequenz (pyc-Gen) verstärkt, insbesondere überexprimiert, wobei man folgende Schritte ausführt:
a) Fermentation der D-Pantothensäure produzierenden Bakterien, in denen zumindest das für Pyruvat-Carboxylase codierende Gen verstärkt wird.
b) Anreicherung der D-Pantothensäure im Medium oder in den Zellen der Bakterien und
c) Isolieren der produzierten D-Pantothensäure.

## Beschreibung

### Stand der Technik

Die Erfindung betrifft ein Verfahren zur fermentativen Herstellung von D-Pantothensäure unter Verwendung coryneformer Bakterien, in denen zumindest das pyc-Gen verstärkt ist.

Die Pantothensäure stellt ein kommerziell bedeutendes Vitamin dar, das in der Kosmetik, der Medizin, der Humanernährung und in der Tierernährung Anwendung findet.

Pantothensäure kann durch chemische Synthese oder biotechnisch durch Fermentation geeigneter Mikroorganismen in geeigneten Nährlösungen hergestellt werden. Bei der chemischen Synthese ist das DL-Pantolacton eine wichtige Zwischenstufe. Es wird in einem mehrstufigen Verfahren aus Formaldehyd, Isobutyraldehyd und Cyanid hergestellt. In weiteren Verfahrensschritten wird das racemische Gemisch aufgetrennt, D-Pantolacton mit β-Alanin kondensiert und so die gewünschte D-Pantothensäure erhalten.

Der Vorteil der fermentativen Herstellung durch Mikroorganismen liegt in der direkten Bildung der gewünschten stereoisomeren D-Form, die frei von L-Pantothensäure ist.

Verschiedene Arten von Bakterien, wie z. B. Escherichia coli, Arthrobacter ureafaciens, Corynebacterium erythrogenes, Brevibacterium ammoniagenes und auch Hefen, wie z. B. Debaromyces castellii können wie in EP-A 0 493 060 gezeigt, in einer Nährlösung, die Glucose, DL-Pantoinsäure und β-Alanin enthält, D-Pantothensäure produzieren. EP-A 0 493 060 zeigt weiterhin, daß bei Escherichia coli durch Amplifikation von Pantothensäure-Biosynthesegenen aus E.coli, die auf den Plasmiden pFV3 und pFV5 enthalten sind, in einer Nährlösung, die Glucose, DL-Pantoinsäure und β-Alanin enthält, die Bildung von D-Pantothensäure verbessert wird.

EP-A 0 590 857 und US-Patent 5,518,906 beschreiben von Escherichia coli Stamm IFO3547 abgeleitete Mutanten, wie FV5714, FV525, FV814, FV521, FV221, FV6051 und FV5069, die Resistenzen gegen verschiedene Antimetabolite wie Salizylsäure, α-Ketobuttersäure, β-Hydroxyasparaginsäure, O-Methylthreonin und α-Ketoisovaleriansäure tragen. Sie produzieren in einer Nährlösung, die Glucose enthält, Pantoinsäure, und in einer Glucose- und β-Alanin-haltigen Nährlösung D-Pantothensäure. In EP-A 0 590 857 und US-Patent 5,518,906 wird weiterhin gezeigt, daß nach Amplifikation der Pantothensäure-Biosynthesegene, die auf dem Plasmid pFV31 enthalten sind, in den oben genannten Stämmen in glucose-haltigen Nährlösungen, die Produktion von D-Pantoinsäure und in einer Nährlösung, die Glucose und β-Alanin enthält, die Produktion von D-Pantothensäure verbessert wird.

### Aufgabe der Erfindung

Die Erfinder haben sich die Aufgabe gestellt, neue Grundlagen für verbesserte Verfahren zur fermentativen Herstellung von Pantothensäure mit coryneformen Bakterien bereitzustellen.

### Beschreibung der Erfindung

Das Vitamin Pantothensäure stellt ein kommerziell bedeutendes Produkt dar, das in der Kosmetik, der Medizin, der Humanernährung und in der Tierernährung Anwendung findet. Es besteht ein allgemeines Interesse daran, verbesserte Verfahren zur Herstellung von Pantothensäure bereitzustellen.

Wenn im Folgenden D-Pantothensäure oder Pantothensäure oder Pantothenat erwähnt werden, sind damit nicht nur die freien Säuren, sondern auch die Salze der D-Pantothensäure wie z.B. das Calcium-, Natrium-, Ammonium- oder Kaliumsalz gemeint.

Gegenstand der Erfindung ist ein Verfahren zur fermentativen Herstellung von D-Pantothensäure unter Verwendung von coryneformen Bakterien, in denen die für das Enzym Pyruvat-Carboxylase (EC-Nummer 6.4.1.1.) kodierende Nucleotidsequenz (pyc-Gen) verstärkt, insbesondere überexprimiert wird.

Die eingesetzten Stämme produzieren gegebenenfalls bereits vor der Verstärkung des pyc-Gens D-Pantothensäure.

Bevorzugte Ausführungsformen finden sich in den Ansprüchen.

Der Begriff 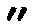 Verstärkung" beschreibt in diesem Zusammenhang die Erhöhung der intrazellulären Aktivität eines oder mehrerer Enzyme in einem Mikroorganismus, die durch die entsprechende DNA kodiert werden, indem man beispielsweise die Kopienzahl des Gens bzw. der Gene erhöht, einen starken Promotor verwendet oder ein Gen verwendet, das für ein entsprechendes Enzym mit einer hohen Aktivität kodiert und gegebenenfalls diese Maßnahmen kombiniert.

Die Mikroorganismen, die Gegenstand der vorliegenden Erfindung sind, können D-Pantothensäure aus Glucose, Saccharose, Lactose, Fructose, Maltose, Melasse, Stärke, Cellulose oder aus Glycerin und Ethanol herstellen. Es handelt sich um Vertreter coryneformer Bakterien, insbesondere der Gattung Corynebacterium. Bei der Gattung Corynebacterium ist insbesondere die Art Corynebacterium glutamicum zu nennen, die in der Fachwelt für ihre Fähigkeit bekannt ist, L-Aminosäuren zu produzieren.

Geeignete Stämme der Gattung Corynebacterium, insbesondere der Art Corynebacterium glutamicum, sind beispielsweise die bekannten Wildtypstämme
Corynebacterium glutamicum ATCC13032
Corynebacterium acetoglutamicum ATCC15806
Corynebacterium acetoacidophilum ATCC13870
Corynebacterium thermoaminogenes FERM BP-1539
Brevibacterium flavum ATCC14067
Brevibacterium lactofermentum ATCC13869 und
Brevibacterium divaricatum ATCC14020
und daraus hergestellte, D-Pantothensäure produzierende Mutanten.

Es wurde gefunden, daß coryneforme Bakterien nach Überexpression des für die Pyruvat-Carboxylase (EC-Nummer 6.4.1.1.) kodierenden pyc-Gens in verbesserter Weise Pantothensäure produzieren.

Das pyc-Gen codiert für das Enzym Pyruvat-Carboxylase (EC-Nummer 6.4.1.1.), welches die Carboxylierung von Pyruvat zu Oxalacetat katalysiert. Die Nukleotidsequenz des pyc-Gens ist in der DE-A-198 31 609 offengelegt und wurde ebenfalls von Koffas et al. (Applied Microbiology and Biotechnology 50, 346-352 (1998)) und Peters-Wendisch et al. (Microbiology 144, 915-927 (1998)) beschrieben. Sie ist bei der Nukleotidsequenzdaten-Bank des National Center for Biotechnology Information (NCBI, Bethesda, MD, USA) unter der accession number Y09548 allgemein verfügbar. Das in den angegebenen Textstellen beschriebene pyc-Gen kann erfindungsgemäß verwendet werden. Weiterhin können Allele des pyc-Gens verwendet werden, die sich aus der Degeneriertheit des genetischen Codes oder durch funktionsneutrale Sinnmutationen (sense mutations) ergeben.

Zur Erzielung einer Verstärkung (z. B. Überexpression) erhöht man z. B. die Kopienzahl der entsprechenden Gene oder mutiert die Promotor- und Regulationsregion oder die Ribosomenbindungsstelle, die sich stromaufwärts des Strukturgens befindet. In gleicher Weise wirken Expressionskassetten, die stromaufwärts des Strukturgens eingebaut werden. Durch induzierbare Promotoren ist es zusätzlich möglich, die Expression im Verlaufe der fermentativen Pantothensäure-Bildung zu steigern. Durch Maßnahmen zur Verlängerung der Lebensdauer der m-RNA wird ebenfalls die Expression verbessert. Weiterhin wird durch Verhinderung des Abbaus des Enzymproteins ebenfalls die Enzymaktivität verstärkt. Die Gene oder Genkonstrukte liegen dabei entweder in Plasmiden mit unterschiedlicher Kopienzahl vor oder sind im Chromosom integriert und amplifiziert. Alternativ kann weiterhin eine Überexpression der betreffenden Gene durch Veränderung der Medienzusammensetzung und Kulturführung erreicht werden.

Anleitungen hierzu findet der Fachmann unter anderem bei Martin et al. (Bio/Technology 5, 137-146 (1987)), bei Guerrero et al. (Gene 138, 35-41 (1994)), Tsuchiya und Morinaga (Bio/Technology 6, 428-430 (1988)), bei Eikmanns et al. (Gene 102, 93-98 (1991)), in der Europäischen Patentschrift EPS 0 472 869, im US Patent 4,601,893, bei Schwarzer und Pühler (Bio/Technology 9, 84-87 (1991), bei Remscheid et al. (Applied and Environmental Microbiology 60, 126-132 (1994)), bei LaBarre et al. (Journal of Bacteriology 175, 1001-1007 (1993)), in der Patentanmeldung WO 96/15246, bei Malumbres et al. (Gene 134, 15-24 (1993)), in der japanischen Offenlegungsschrift JP-A-10-229891, bei Jensen und Hammer (Biotechnology and Bioengineering 58, 191-195 (1998)) und in bekannten Lehrbüchern der Genetik und Molekularbiologie.

Ein Beispiel für ein Plasmid mit Hilfe dessen die Pyruvat-Carboxylase überexprimiert wird, ist das Plasmid pVWEx1pyc, das in der DE-A-198 31 609 offengelegt ist und auf dem Ausgangsvektor pVWEx1 beruht. Plasmid pVWEx1 enthält als Bestandteile unter anderem Sequenzen des Plasmids pBL1, den tac-Promotor und das lacI^{q}-Allel. Andere in C. glutamicum replizierbare Plasmidvektoren, wie z.B. pEKEx1 (Eikmanns et al., Gene 102:93-98 (1991)) oder pZ8-1 (Europäische Patentschrift 0 375 889), können in gleicher Weise verwendet werden.

Zusätzlich kann es für die Produktion von Pantothensäure vorteilhaft sein, neben dem für die Pyruvat-Carboxylase codierende Gen ein oder mehrere weitere für Enzyme des Pantothensäure-Biosyntheseweges oder des Keto-Isovaleriansäure-Biosyntheseweges codierende Gene wie z. B.
- das für die Aspartat-Decarboxylase kodierende panD-Gen (Dusch et al., Applied and Environmental Microbiology 65, 1530-1539 (1999)) oder
- das für die Ketopantoat-Hydroxymethyltransferase kodierende panB-Gen (Sahm et al., Applied and Environmental Microbiology, 65, 1973-1979 (1999)) oder
- das für die Pantothenat-Synthetase kodierende panC-Gen (Sahm et al., Applied and Environmental Microbiology, 65, 1973-1979 (1999)) oder
- das für die Dihydroxysäure-Dehydratase kodierende ilvD-Gen
zu verstärken, insbesondere zu überexprimieren.

Weiterhin kann es für die Produktion von Pantothensäure vorteilhaft sein, neben der Überexpression der Pyruvat-Carboxylase unerwünschte Nebenreaktionen auszuschalten (Nakayama: 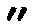 Breeding of Amino Acid Producing Microorganisms 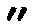 , in: Overproduction of Microbial Products, Krumphanzl, Sikyta, Vanek (eds.), Academic Press, London, UK, 1982).

Die erfindungsgemäß hergestellten Mikroorganismen können kontinuierlich oder diskontinuierlich im batch - Verfahren (Satzkultivierung) oder im fed batch (Zulaufverfahren) oder repeated fed batch Verfahren (repetitives Zulaufverfahren) zum Zwecke der Pantothensäure-Produktion kultiviert werden.

Eine Zusammenfassung über bekannte Kultivierungsmethoden ist im Lehrbuch von Chmiel (Bioprozesstechnik 1. Einführung in die Bioverfahrenstechnik (Gustav Fischer Verlag, Stuttgart, 1991)) oder im Lehrbuch von Storhas (Bioreaktoren und periphere Einrichtungen (Vieweg Verlag, Braunschweig/Wiesbaden, 1994)) beschrieben.

Das zu verwendende Kulturmedium muß in geeigneter Weise den Ansprüchen der jeweiligen Mikroorganismen genügen. Beschreibungen von Kulturmedien verschiedenener Mikroorganismen sind im Handbuch "Manual of Methods for General Bacteriology" der American Society for Bacteriology (Washington D.C., USA, 1981) enthalten. Als Kohlenstoffquelle können Zucker und Kohlehydrate wie z.B. Glucose, Saccharose, Lactose, Fructose, Maltose, Melasse, Stärke und Cellulose, Öle und Fette wie z. B. Sojaöl, Sonnenblumenöl, Erdnussöl und Kokosfett, Fettsäuren wie z. B. Palmitinsäure, Stearinsäure und Linolsäure, Alkohole wie z. B. Glycerin und Ethanol und organische Säuren wie z. B. Essigsäure verwendet werden. Diese Stoffe können einzeln oder als Mischung verwendet werden. Als Stickstoffquelle können organische, Stickstoff-haltige Verbindungen wie Peptone, Hefeextrakt, Fleischextrakt, Malzextrakt, Maisquellwasser, Sojabohnenmehl und Harnstoff oder anorganische Verbindungen wie Ammoniumsulfat, Ammoniumchlorid, Ammoniumphosphat, Ammoniumcarbonat und Ammoniumnitrat verwendet werden. Die Stickstoffquellen können einzeln oder als Mischung verwendet werden. Als Phosphorquelle können Kaliumdihydrogenphosphat oder Dikaliumhydrogenphosphat oder die entsprechenden Natriumhaltigen Salze verwendet werden. Das Kulturmedium muß weiterhin Salze von Metallen enthalten wie z.B. Magnesiumsulfat oder Eisensulfat, die für das Wachstum notwendig sind. Schließlich können essentielle Wuchsstoffe, wie Aminosäuren und Vitamine zusätzlich zu den oben genannten Stoffen eingesetzt werden. Dem Kulturmedium können überdies zur zusätzlichen Steigerung der Pantothensäure-Produktion Vorstufen der Pantothensäure, wie Aspartat, β-Alanin, Ketolsovalerat, Ketopantoinsäure oder Pantoinsäure, und gegebenenfalls deren Salze zugesetzt werden. Die genannten Einsatzstoffe können zur Kultur in Form eines einmaligen Ansatzes hinzugegeben oder in geeigneter Weise während der Kultivierung zugefüttert werden.

Zur pH - Kontrolle der Kultur werden basische Verbindungen wie Natriumhydroxid, Kaliumhydroxid, Ammoniak oder saure Verbindungen wie Phosphorsäure oder Schwefelsäure in geeigneter Weise eingesetzt. Zur Kontrolle der Schaumentwicklung können Antischaummittel wie z.B. Fettsäurepolyglykolester eingesetzt werden. Zur Aufrechterhaltung der Stabilität von Plasmiden können dem Medium geeignete selektiv wirkende Stoffe, z.B. Antibiotika, hinzugefügt werden. Um aerobe Bedingungen aufrechtzuerhalten werden Sauerstoff oder Sauerstoffhaltige Gasmischungen wie z.B. Luft in die Kultur eingetragen. Die Temperatur der Kultur liegt normalerweise bei 20°C bis 45°C und vorzugsweise bei 25°C bis 40°C. Die Kultur wird solange fortgesetzt bis sich ein Maximum an Pantothensäure gebildet hat. Dieses Ziel wird normalerweise innerhalb von 10 Stunden bis 160 Stunden erreicht.

Die Konzentration an gebildeter Pantothensäure kann mit bekannten Verfahren (Velisek; Chromatographic Science 60, 515-560 (1992)) bestimmt werden.

Folgender Mikroorganismus wurde bei der Deutschen Sammlung für Mikroorganismen und Zellkulturen (DSMZ, Braunschweig, Deutschland) gemäß Budapester Vertrag 1. Juli 1999 hinterlegt:

Corynebacterium glutamicum DG52-5/pVWEx1pyc als DSM12893

### Beispiele

Die vorliegende Erfindung wird im folgenden anhand von Ausführungsbeispielen näher erläutert.

Zu diesem Zweck wurden Versuche mit dem Isoleucinbedürftigen Stamm ATCC13032ΔilvA durchgeführt, der als DSM12455 bei der Deutschen Sammlung für Mikroorganismen und Zellkulturen in Braunschweig (Deutschland) gemäss Budapester Vertrag hinterlegt worden ist.

### Beispiel 1

### Herstellung des Stammes ATCC13032ΔilvA/pVWEx1pyc

Durch Elektroporation (Tauch et.al., 1994, FEMS Microbiological Letters, 123:343-347) des Plasmids pVWEx1pyc (DE-A-198 31 609) in den C. glutamicum Stamm ATCC13032ΔilvA und anschließender Selektion auf LB-Agar (Lennox, 1955, Virology, 1:190-206) + 25 µg/ml Kanamycin wurde der Stamm ATCC13032ΔilvA/pVWEx1pyc erhalten.

### Beispiel 2

### Herstellung von Pantothensäure

Die Bildung von Pantothenat durch die C. glutamicum Stämme ATCC13032ΔilvA und ATCC13032ΔilvA/pVWEx1pyc wurde in Medium CGXII (Keilhauer et al., 1993, Journal of Bacteriology, 175:5595-5603; Tabelle 4), das mit 25 µg/ml Kanamycin und 25 µg/ml Isoleucin supplementiert wurde, geprüft. Dieses Medium wird im Folgenden als C. glutamicum-Testmedium bezeichnet. Je 50 ml frisch angesetztes C. glutamicum-Testmedium wurden aus einer 16 Stunden alten Vorkultur des gleichen Mediums dergestalt angeimpft, daß die optische Dichte der Kultursuspension (o.D.₅₈₀) bei Inkubationsbeginn 0,1 betrug. Nach 24stündiger Inkubation bei 30°C und 175 U/min wurde die optische Dichte (o.D.₅₈₀) der Kultur bestimmt und anschließend die Zellen durch 10minütige Zentrifugation bei 5000 g entfernt und der Überstand sterilfiltriert.

Zur Bestimmung der optischen Dichte wurde ein Novaspec II Photometer der Firma Pharmacia (Freiburg, Deutschland) bei einer Messwellenlänge von 580 nm eingesetzt.

Die Quantifizierung des D-Pantothenats im Kulturüberstand erfolgte mittels Lactobacillus plantarum ATCC 8014 nach Angaben des Handbuchs der Firma DIFCO (DIFCO MANUAL, 10^{th} Edition, S. 1100-1102; Michigan, USA). Für die Kalibrierung wurde das Hemicalciumsalz von Pantothenat der Firma Sigma (Deisenhofen, Deutschland) verwendet.

Die Ergebnisse der Pantothenat-Produktion durch die Stämme ATCC13032ΔilvA und ATCC13032ΔilvA/pVWEx1pyc sind in Tabelle 1 zusammengefasst.

**Tabelle 1**

| Stamm | Gen | Zelldichte oD₅₈₀ | Konzentration (ng/ml) |
|---|---|---|---|
| ATCC13032ΔilvA | - | 11,3 | 3,8 |
| ATCC13032ΔilvA/pVWEx1pyc | pyc | 9,3 | 8,5 |

### Beispiel 3

### Herstellung des Plasmides pXT-panD

### 3.1 Herstellung des E. coli - C. glutamicum Pendelvektors pEC-XT99A

Als Ausgangsvektor zur Konstruktion des E. coli-C. glutamicum-Shuttle-Expressionsvektors pEC-XT99A wurde der E.coli-Expressionsvektor pTRC99A (Amann et al. 1988, Gene 69:301-315) verwandt. Nach BspHI-Restriktionsspaltung (Roche Diagnostics GmbH, Mannheim, Deutschland, Produktbeschreibung BspHI, Product No. 1467123) und anschließender Klenow-Behandlung (Amersham Pharmacia Biotech, Freiburg, Deutschland, Produktbeschreibung Klenow Fragment of DNA Polymerase I, Product No. 27-0928-01; Methode nach Sambrook et al., 1989, Molecular Cloning: A laboratory Manual, Cold Spring Harbor) wurde das Ampicillin-Resistenzgen (bla) gegen das Tetracyclin-Resistenzgen des C. glutamicum Plasmids pAG1 (GenBank Accession No. AF121000) ausgetauscht. Hierzu wurde der Resistenzgen-tragende Bereich als AluI-Fragment (Amersham Pharmacia Biotech, Freiburg, Deutschland, Produktbeschreibung AluI, Product No. 27-0884-01) in den linearisierten E.coli-Expressionsvektor pTRC99A kloniert. Die Ligation wurde wie von Sambrook et al. (1989, Molecular Cloning: A laboratory Manual, Cold Spring Harbor) beschrieben durchgeführt, wobei das DNA-Gemisch mit T4-Ligase (Amersham Pharmacia Biotech, Freiburg, Deutschland, Produktbeschreibung T4-DNA-Ligase, Product No. 27-0870-04) über Nacht inkubiert wurde. Dieses Ligationsgemisch wurde anschließend in den E. coli-Stamm DH5αmcr (Grant, 1990, Proceedings of the National Academy of Sciences U.S.A., 87:4645-4649) elektroporiert (Tauch et al. 1994, FEMS Microbiology Letters, 123:343-7). Der konstruierte E. coli-Expressionsvektor wurde mit pXT99A bezeichnet.

Als Basis zur Klonierung eines Minimalreplikons aus Corynebacterium glutamicum wurde das Plasmid pGA1 (Sonnen et al. 1991, Gene, 107:69-74) verwandt. Durch BalI/PstI-Restriktionsspaltung (Promega GmbH, Mannheim, Deutschland, Produktbeschreibung BalI, Product No. R6691; Amersham Pharmacia Biotech, Freiburg, Deutschland Produktbeschreibung PstI, Product No. 27-0976-01) des Vektors pGA1 konnte ein 3484 bp großes Fragment in den mit SmaI und PstI (Amersham Pharmacia Biotech, Freiburg, Deutschland, Produktbeschreibung SmaI, Product No. 27-0942-02, Produktbeschreibung PstI, Product Na. 27-0976-01) fragmentierten Vektor pK18mob2 (Tauch et al., 1998, Archives of Microbiology 169:303-312) kloniert werden. Mittels BamHI/XhoI-Restriktionsspaltung (Amersham Pharmacia Biotech, Freiburg, Deutschland, Produktbeschreibung BamHI, Product No. 27-086803, Produktbeschreibung XhoI, Product No. 27-0950-01) und anschließender Klenow-Behandlung (Amersham Pharmacia Biotech, Freiburg, Deutschland, Produktbeschreibung Klenow Fragment of DNA Polymerase I, Product No. 27-0928-01; Methode nach Sambrook et al., 1989, Molecular Cloning: A laboratory Manual, Cold Spring Harbor) wurde ein 839 bp großes Fragment deletiert. Aus dem mit T4-Ligase (Amersham Pharmacia Biotech, Freiburg, Deutschland, Produktbeschreibung T4-DNA-Ligase, Product No. 27-0870-04) religierten Konstrukt konnte das C. glutamicum Minimalreplikon als 2645 bp großes Fragment in den E.coli-Expressionsvektor pXT99A kloniert werden. Hierzu wurde die DNA des Minimalreplikon-tragenden Konstruktes mit den Restriktionsenzymen KpnI (Amersham Pharmacia Biotech, Freiburg, Deutschland, Produktbeschreibung KpnI, Product No. 27-0908-01) und PstI (Amersham Pharmacia Biotech, Freiburg, Deutschland, Produktbeschreibung PstI, Product No. 27-0886-03) gespalten und anschließend mittels Klenow-Polymerase (Amersham Pharmacia Biotech, Freiburg, Deutschland, Produktbeschreibung Klenow Fragment of DNA Polymerase I, Product No. 27-0928-01) eine 3'-5'-Exonukleasebehandlung (Sambrook et al., 1989, Molecular Cloning: A laboratory Manual, Cold Spring Harbor) durchgeführt.

In einem parallelen Ansatz wurde der E.coli-Expressionsvektor pXT99A mit dem Restriktionsenzym RsrII (Roche Diagnostics, Mannheim, Deutschland, Produktbeschreibung RsrII, Product No. 1292587) gespalten und mit Klenow-Polymerase (Amersham Pharmacia Biotech, Freiburg, Deutschland, Klenow Fragment of DNA Polymerase I, Product No. 27-0928-01) zur Ligation vorbereitet. Die Ligation des Minimalreplikons mit dem Vektorkonstrukt pXT99A wurde wie von Sambrook et al. (1989, Molecular Cloning: A laboratory Manual, Cold Spring Harbor) beschrieben durchgeführt, wobei das DNA-Gemisch mit T4-Ligase (Amersham Pharmacia Biotech, Freiburg, Deutschland, Produktbeschreibung T4-DNA-Ligase, Product No. 27-0870-04) über Nacht inkubiert wurde.

Der so konstruierte E. coli-C. glutamicum-Shuttle-Expressionsvektor pEC-XT99A wurde mittels Elektroporation (Liebl et al., 1989, FEMS Microbiology Letters, 53:299-303) in C. glutamicum DSM5715 transferiert. Die Selektion der Transformanten erfolgte auf LBHIS Agar bestehend aus 18,5 g/l Brain-Heart Infusion Boullion, 0,5 M Sorbitol, 5 g/l Bacto-Trypton, 2,5 g/l Bacto-Yeast-Extract, 5 g/l NaCl und 18 g/l Bacto-Agar, der mit 5 mg/l Tetracyclin supplementiert worden war. Die Inkubation erfolgte für 2 Tage bei 33°C.

Plasmid DNA wurde aus einer Transformante nach den üblichen Methoden isoliert (Peters-Wendisch et al., 1998, Microbiology, 144, 915 - 927), mit der Restriktionsendonuklease HindIII geschnitten und das Plasmid durch anschließende Agarosegel-Elektrophorese überprüft.

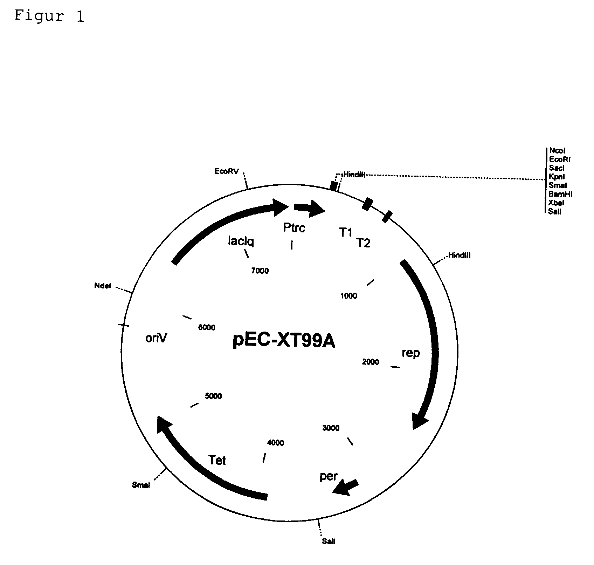

Das so erhaltene Plasmidkonstrukt wurde als pEC-XT99A bezeichnet und ist in Figur 1 dargestellt. Der durch Elektroporation des Plasmides pEC-XT99A in den Corynebacterium glutamicum Stamm DSM5715 erhaltene Stamm wurde DSM5715/pEC-XT99A genannt und als DSM12967 bei der Deutschen Sammlung für Mikroorganismen und Zellkulturen (DSMZ, Braunschweig, Deutschland) gemäß Budapester Vertrag hinterlegt.

### 3.2 Herstellung des Plasmides pXT-panD

Ausgehend von den Nucleotidsequenzen des Pantothenatbiosynthese Gens panD von C. glutamicum ATCC 13032 (Dusch et al. (Applied and Environmental Microbiology 65(4), 1530-1539 (1999)) und DE 19855313.7) wurden PCR-Primer so ausgewählt, daß das amplifizierte Fragment das Gen mit seiner nativen Ribosomen-Bindestellen enthält. Das mit den PCR-Primer panD-Cg1 (5'-CATCTCACGCTATGAATTCT-3') und panD-Cg2 (5'-ACGAGGCCTGCAGCAATA-3) amplifizierte 405 bp große Fragment wurde den Herstellerangaben zufolge in den Vektor pCR®2.1 (Original TA Cloning Kit, Invitrogene (Leek, Niederlande), Produktbeschreibung Original TA Cloning® Kit, Cat. no. KNM2030-01) ligiert und anschließend in den E. coli Stamm TOP10F'(Katalog 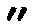 Invitrogen 2000" der Firma Invitrogen, Groningen, Niederlande) transformiert. Die Selektion auf Transformanten erfolgte durch Inkubation bei 37°C für 24 Stunden auf LB-Agarplatten mit 100 µg/ml Ampicillin und 40 µg/ml X-Gal (5-Bromo-4-Chloro-3-Indolyl-β-D-Galaktosid).

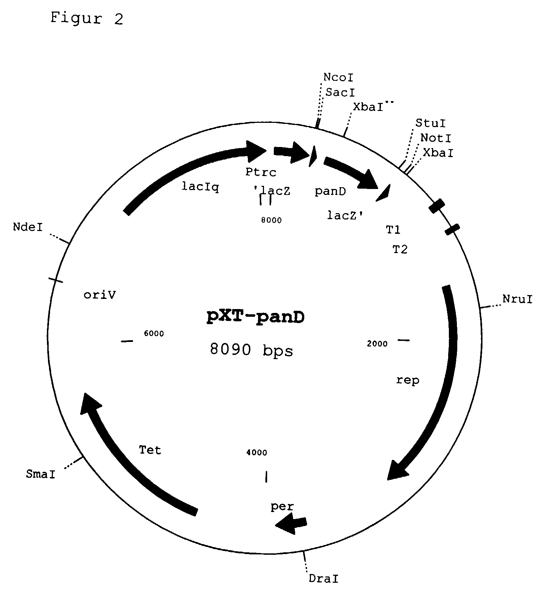

DNA des so konstruierten Plasmids pCR-D2 wurde nach der üblichen Methode aus einer Transformante isoliert, mit den Restriktionsendonukleasen SacI und XbaI verdaut und in den ebenso gespaltenen Vektor pEC-XT99A ligiert. Da die zweite XbaI-Schnittstelle, welche in der panD-Kodierregion liegt, in dem E. coli Wirt Top10F 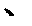 methyliert vorliegt, wurde diese Spaltstelle nicht geschnitten und das Gen wurde folglich intakt durch die flankierenden SacI und XbaI Schnittstellen aus dem Plasmid pCR-D2 herausgespalten. Nach der Ligation wurde der Ansatz in den Stamm E. coli DH5αmcr elektroporiert. Die Selektion erfolgte auf LB-Agarplatten mit 50 µg/ml Kanamycin. Plasmid-DNA aus einer so erhaltenen Transformante wurde isoliert, mit den Restriktionsendonukleasen SacI und XbaI gespalten und die Fragmente wurden anschließend durch Agarose-Gelelektrophorese überprüft. Das so konstruierte Plasmid wurde als pXT-panD bezeichnet und ist in Figur 2 dargestellt.

### Beispiel 4

### Herstellung des Pantothensäure-Produzenten ATCC13032ΔilvA/pVWEx1pyc,pXT-panD

Das in Beispiel 3 beschriebene Plasmid pXT-panD wurde in den C. glutamicum Stamm ATCC13032ΔilvA/pVWEx1pyc elektroporiert. Nach zweitägiger Selektion bei 30°C auf LB-Agar, der mit 10 µg/ml Tetracyclin und 25 µg/ml Kanamycin supplementiert worden war, wurde der Stamm ATCC13032ΔilvA/pVWEx1pyc,pXT-panD erhalten.

### Beispiel 5

### Herstellung von Pantothensäure

Die Bildung von Pantothenat durch die C. glutamicum Stämme ATCC13032ΔilvA/pVWEx1pyc,pXT-panD und ATCC13032ΔilvA/pXT-panD wurde in Medium CGXII (Keilhauer et al., 1993, Journal of Bacteriology, 175:5595-5603; Tabelle 2) geprüft, das mit 10 µg/ml Tetracyclin, 2 mM Isoleucin und im Falle des Stammes ATCC13032ΔilvA/pVWEx1pyc,pXT-panD mit zusätzlich 25 µg/ml Kanamycin supplementiert worden war.

Dieses Medium wird im Folgenden als C. glutamicum-Testmedium bezeichnet. Je 50 ml frisch angesetztes C. glutamicum-Testmedium wurden aus einer 16 Stunden alten Vorkultur des gleichen Mediums dergestalt angeimpft, daß die optische Dichte der Kultursuspension (o.D.₅₈₀) bei Inkubationsbeginn 0,1 betrug. Die Kulturen wurden bei 30°C und 130 U/min bebrütet. Nach 5 stündiger Inkubation wurde IPTG (Isopropyl β-D-thiogalactosid) in einer Endkonzentration von 1 mM hinzugefügt. Nach 48stündiger Inkubation wurde die optische Dichte (o.D.₅₈₀) der Kultur bestimmt und anschließend die Zellen durch 10minütige Zentrifugation bei 5000 g entfernt und der Überstand sterilfiltriert.

Zur Bestimmung der optischen Dichte wurde ein Novaspec II Photometer der Firma Pharmacia (Freiburg, Deutschland) bei einer Messwellenlänge von 580 nm eingesetzt.

Die Quantifizierung des D-Pantothenats im Kulturüberstand erfolgte mittels Lactobacillus plantarum ATCC 8014 nach Angaben des Handbuchs der Firma DIFCO (DIFCO MANUAL, 10^{th} Edition, S. 1100-1102; Michigan, USA). Für die Kalibrierung wurde das Hemicalciumsalz von Pantothenat der Firma Sigma (Deisenhofen, Deutschland) verwendet.

Das Ergebnis ist in Tabelle 3 dargestellt.

**Tabelle 2**

| Substanz | Menge pro Liter | Bemerkung |
|---|---|---|
| (NH₄)₂ SO₂ | 20 g | |
| Harnstoff | 5 g | |
| KH₂PO₄ | 1 g | |
| K₂HPO₄ | 1 g | |
| MgSO₄ * 7 H₂O | 0.25 g | |
| MOPS | 42 g | |
| CaCl₂ | 10 mg | |
| FeSO₄ * 7 H₂O | 10 mg | |
| MnSO₄ * H₂O | 10 mg | |
| ZnSO₄ * 7 H₂O | 1 mg | |
| CuSO₄ | 0.2 mg | |
| NiCl₂ * 6 H₂O | 0.02 mg | |
| Biotin | 0.5 mg | |
| Glukose | 40 g | separat autoklavieren |
| Protocatechusäure | 0.03 mg | sterilfiltrieren |

**Tabelle 3**

| Stamm | Gen | Zelldichte oD₅₈₀ | Konzentration (ng/ml) |
|---|---|---|---|
| ATCC13032ΔilvA/pVWEx1pyc | pyc | 17 | 8,3 |
| ATCC13032ΔilvA/pVWEx1pyc, pXT-panD | pyc, panD | 19 | 172 |

### Abbildungen:

Folgende Figuren sind beigefügt:
- Figur 1:: Restriktionskarte des Plasmids pEC-XT99A
- Figur 2:: Restriktionskarte des Plasmids pXT-panD

Bei der Angabe der Basenpaarzahlen handelt es sich um ca.-Werte, die im Rahmen der Reproduzierbarkeit erhalten werden.

Die verwendeten Abkürzungen und Bezeichnungen haben folgende Bedeutung:
- 'lacZ:: 3'-Terminus des lacZα Genfragmentes
- lacIq:: LacIq Allel des lac Repressorgens
- lacZ':: 5'-Terminus des lacZα Genfragmentes
- oriV:: Replikationsursprung V
- panD:: Aspartat-Decarboxylase Gen
- per:: Gen zur Kontrolle der Kopienzahl
- Ptrc:: trc-Promotor
- rep:: Replikationsregion für C. glutamicum
- T1:: Transkriptionsterminator T1
- T2:: Transkriptionsterminator T2
- Tet:: Tetracyclinresistenzgen
- BamHI:: Schnittstelle des Restriktionsenzyms BamHI
- DraI:: Schnittstelle des Restriktionsenzyms DraI
- EcoRI:: Schnittstelle des Restriktionsenzyms EcoRI
- EcoRV:: Schnittstelle des Restriktionsenzyms EcoRV
- HindIII:: Schnittstelle des Restriktiorisenzyms HindIII
- KpnI:: Schnittstelle des Restriktionsenzyms KpnI
- NdeI: Schnittstelle des Restriktionsenzyms NdeI
- NotI:: Schnittstelle des Restriktionsenzyms NotI
- NruI:: Schnittstelle des Restriktionsenzyms NruI
- PstI:: Schnittstelle des Restriktionsenzyms PstI
- SacI:: Schnittstelle des Restriktionsenzyms SacI
- SalI:: Schnittstelle des Restriktionsenzyms SalI
- SmaI:: Schnittstelle des Restriktionsenzyms SmaI
- XbaI**:: Methylierte Schnittstelle XbaI
- XbaI:: Schnittstelle des Restriktionsenzyms XbaI

## Patentansprüche

1. Verfahren zur Herstellung von D-Pantothensäure durch Fermentation coryneformer Bakterien,
**dadurch gekennzeichnet,**
daß man Bakterien einsetzt, in denen man die für die Pyruvat-Carboxylase (EC-Nummer 6.4.1.1.) codierende Nucleotidsequenz (pyc-Gen) verstärkt, insbesondere überexprimiert.

2. Verfahren gemäß Anspruch 1,
**dadurch gekennzeichnet,**
daß man Bakterien einsetzt, in denen man zusätzlich weitere Gene des Biosyntheseweges der D-Pantothensäure verstärkt.

3. Verfahren gemäß Anspruch 1,
**dadurch gekennzeichnet,**
daß man Bakterien einsetzt, in denen die Stoffwechselwege zumindest teilweise ausgeschaltet sind, die die Bildung der D-Pantothensäure verringern.

4. Verfahren gemäß einem oder mehreren der vorhergehenden Ansprüche,
**dadurch gekennzeichnet,**
daß man einen mit einem Plasmidvektor transformierten Stamm einsetzt, und der Plasmidvektor die für die Pyruvat-Carboxylase codierende Nucleotidsequenz trägt.

5. Verfahren gemäß Anspruch 4,
**dadurch gekennzeichnet,**
daß man mit dem Plasmid pVWEx1pyc transformierte Bakterien einsetzt.

6. Verfahren gemäß Anspruch 1,
**dadurch gekennzeichnet,**
daß man gleichzeitig das für die Aspartat-Decarboxylase kodierende panD-Gen verstärkt.

7. Verfahren gemäß Anspruch 1,
**dadurch gekennzeichnet,**
daß man gleichzeitig das für die Ketopantoat-Hydroxymethyltransferase kodierende panB-Gen verstärkt.

8. Verfahren gemäß Anspruch 1,
**dadurch gekennzeichnet,**
daß man gleichzeitig das für die Pantothenat-Synthetase kodierende panC-Gen verstärkt.

9. Verfahren gemäß Anspruch 1,
**dadurch gekennzeichnet,**
daß man gleichzeitig das für die Dihydroxysäure-Dehydratase kodierende ilvD-Gen verstärkt.

10. Verfahren gemäß einem oder mehreren der vorhergehenden Ansprüche,
**dadurch gekennzeichnet,**
daß man die genannten Gene in coryneformen Bakterien verstärkt, die bereits D-Pantothensäure produzieren.

11. Verfahren zur fermentativen Herstellung von D-Pantothensäure gemäß einem oder mehreren der vorhergehenden Ansprüche,
**dadurch gekennzeichnet,**
daß man folgende Schritte durchführt:
a) Fermentation der D-Pantothensäure produzierenden Bakterien, in denen zumindest das für die Pyruvat-Carboxylase codierende Gen verstärkt wird,
b) Anreicherung der D-Pantothensäure im Medium oder in den Zellen der Bakterien und
c) Isolieren der produzierten D-Pantothensäure.

12. Coryneforme Bakterien, in denen die für die Pyruvat-Carboxylase (EC-Nummer 6.4.1.1.) codierende Nucleotidsequenzen (pyc-Gen) verstärkt, insbesondere überexprimiert sind.

13. Corynebacterium glutamicum DG 52-5/pVWEx1pyc hinterlegt unter der Nummer DSM 12893 bei der DSMZ, Braunschweig.
